(19) Europäisches Patentamt European Patent Office Office européen des brevets

(11) **EP 2 086 310 B1**

(12) **EUROPEAN PATENT SPECIFICATION**

(45) Date of publication and mention of the grant of the patent:
**03.10.2018 Bulletin 2018/40**

(21) Application number: **07835199.6**

(22) Date of filing: **13.11.2007**

(51) Int Cl.:
*A01J 5/013* (2006.01)    *G01N 33/04* (2006.01)

(86) International application number:
**PCT/SE2007/000999**

(87) International publication number:
**WO 2008/066440 (05.06.2008 Gazette 2008/23)**

(54) **METHOD FOR DETECTING MASTITIS OF MILKING ANIMALS, A MILKING SYSTEM AND A COMPUTER PROGRAM PRODUCT**

VERFAHREN ZUM NACHWEIS VON MASTITIS BEI MILCHTIEREN, MELKSYSTEM UND COMPUTERPROGRAMMPRODUKT

PROCÉDÉ PERMETTANT DE DIAGNOSTIQUER UNE MASTITE CHEZ LES ANIMAUX À TRAIRE, SYSTÈME DE TRAITE ET PRODUIT DE PROGRAMME INFORMATIQUE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **30.11.2006 SE 0602565**

(43) Date of publication of application:
**12.08.2009 Bulletin 2009/33**

(73) Proprietor: **DeLaval Holding AB 147 21 Tumba (SE)**

(72) Inventors:
• **ÖHMAN, Ulrika
129 40 Hägersten (SE)**

• **MARKUSSON, Ola
141 37 Huddinge (SE)**

(74) Representative: **Lilliehorn, Tobias et al
DeLaval International AB
Intellectual Property Support
Legal Affairs
P.O.Box 39
147 21 Tumba (SE)**

(56) References cited:
| EP-A2- 1 340 457 | WO-A1-01/56369 |
| WO-A1-97/47187 | WO-A1-99/01026 |
| WO-A1-2005/006849 | WO-A2-00/39578 |
| US-A1- 2002 124 803 | |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the invention

The present invention relates generally to the field of milk producing animals, and in particular to an improved method for detecting mastitis of a milking animal.

### Background of the invention

The health of milking animals, such as cows, is of utmost importance in a milking farm. Mastitis is a rather common infection among cows and usually affects individual teats of the udder, although the entire udder may be affected. Mastitis may be painful for the animal and also degrades the quality of the milk.

Mastitis in a herd of milking animals causes economic loss for the farmer due to treatment costs, lost quarters, reduced milk production and milk having to be discarded for not fulfilling quality requirements. It is therefore important to prevent mastitis to the largest possible extent, and to detect occurrences of it as soon as possible.

There are different ways to detect mastitis. The farmer may for example inspect the milk and/or the udder visually, perform conductivity measurements, measure the temperature of the milk or measure the milk flow or the quantity of milk from the different teats.

Document WO01/563609 A1 describes a method where an animal is diagnosed with mastitis based on two different indicators, which could be the color and conductivity of milk.

All these methods only give rough indications of mastitis and are thus not fully reliable. For example, the result from a conductivity test may be affected by the amount of water that the animal has been drinking and on her current salt level when the test is performed. Further, some of the methods, for example a visual inspection, are also time-consuming and therefore expensive.

California Mastitis Test (CMT) is another example of a method for detecting mastitis. It is based on the knowledge that the number of somatic cells increases if the animal is infected by mastitis. A reagent is added to a sample of the milk being tested and interacts with the DNA and proteins in the somatic cells to form a gel. The viscosity of the gel is then measured and used as an indication of a somatic cell concentration, wherein a high viscosity may be an indication of mastitis.

However, the CMT is not an actual cell counting, but only an estimate of within which interval the number of cells presumably lies, which lowers the accuracy of the test. Further, the CMT is usually performed manually, which manual handling may also affect the accuracy of the test.

Cell counting is one of the most reliable indicators of mastitis. In cell counting the number of somatic cells, and in particular white blood cells, is counted. When a farmer wishes to make a cell count, a milk sample is taken manually and sent to a laboratory for analysis. The result is then received several weeks later and such handling is obviously both very time-consuming and expensive and is typically performed only once a month for each animal. Further, since the results are not obtained immediately, mastitis treatment is delayed.

An improvement in this regard is on-line cell counters, by means of which a daily monitoring of the somatic cell count level and trends is enabled.

Although cell counting is a reliable method, there are nevertheless some drawbacks when using it as an indicator of mastitis, the costs of performing cell counts being a major disadvantage.

All the above-mentioned methods for detecting mastitis thus have drawbacks. It would therefore be desirable to provide an in many aspects improved method for detecting mastitis of milking animals in a milking farm.

### Summary of the invention

It is an object of the invention to provide an improved method for detecting mastitis among milking animals for overcoming or at least alleviating shortcomings of the prior art. In particular, it is an object of the invention to provide a most reliable method for detecting mastitis among the milking animals, giving an early indication of diseased animals.

It is another object of the invention to provide a method for detecting mastitis, wherein the costs related to mastitis detection can be minimized.

It is yet another object of the invention to provide a method for detecting mastitis that is easily implemented in existing milking systems.

These objects, among others, are achieved by a method, by a milking system, by a computer program product stored on a computer readable storage medium and by a computer program product directly loadable into the internal memory of a digital computer as claimed in the independent claims.

In accordance with the invention a method is provided for detecting mastitis of a milking animal. The method comprises the steps of: determining a first indicator of mastitis; determining a second indicator of mastitis; evaluating the first and the second indicators of mastitis; and identifying a need for a third indicator of mastitis if the step of evaluating indicates a need for such third indicator of mastitis. An increased reliability is obtained by the method, while the costs are still kept at a minimum.

All the steps of the method may be performed automatically. This provides a very fast and reliable way of detecting mastitis within a herd of milking animals. The costs are minimized, as well as the time required to perform the tests.

In accordance with an embodiment of the invention, the third indicator of mastitis is a somatic cell count value, which gives a very reliable indication of mastitis. By means of the invention, the costs for somatic cell count tests can be minimized, since such a test is performed only if at least two more easily obtainable indicators of mastitis indicate a need for a somatic cell count. The somatic cell count is thus performed only when needed, whereby the time required to perform such test as well as the related costs can be kept at a minimum. The first and the second indicators of mastitis are preferably obtained by cheap and readily performed tests, whereby they may be done at each milking occasion. An early detection of mastitis can thereby be accomplished. Further, the method is easily implemented in existing milking systems, since existing equipment may be utilized.

The step of determining the first indicator may comprise measuring the conductivity of the milk from a first teat of the milking animal and the step of determining the second indicator may comprise measuring the conductivity of the milk from a second teat. The step of evaluating may then comprise comparing the conductivity value of milk from the first teat with the conductivity value of milk from the second teat. If the comparison shows a difference that is above a threshold value, then a need for a third indicator of mastitis is identified. An implementation of the invention that is easily implemented is provided, which provides a fast and cost-efficient way to decide on whether or not to perform a third mastitis detection test.

The step of determining the first indicator of mastitis and/or the step of determining the second indicator of mastitis may instead comprise determining colour changes in the milk.

In accordance with another embodiment of the invention, the third indicator of mastitis is instead obtained by a bacterial investigation and/or an examination by a veterinary. In this embodiment, the first and/or the second indicator of mastitis is preferably a somatic cell count value. The combination of the cell count value with historical data about old cell count values, milk yield, previous treatments etc. gives a very reliable indication of mastitis, but there is still a need for a further investigation in order to determine the type of mastitis and the need for treatment. A bacterial investigation and/or an examination by a veterinary is thus performed only when needed, and the related costs can be kept at a minimum. A somatic cell count test, even though expensive compared to cheaper tests like measuring the conductivity of the milk or determining colour changes in the milk, is still much cheaper than a bacterial investigation or an examination by a veterinary.

The invention is also related to a corresponding milking system, whereby advantages similar to the above are achieved. The invention further relates to a computer program product stored on a computer readable storage medium and a computer program product directly loadable into the internal memory of a digital computer.

Further characteristics of the invention and advantages thereof will be evident from the detailed description of embodiments of the present invention given hereinafter and the accompanying figures, which are only given by way of illustration.

## Brief description of the drawings

Figure 1 illustrates an automatic milking system in which the present invention may be implemented.

Figure 2 is a flow chart over the steps of a method in accordance with the invention.

## Detailed description of embodiments

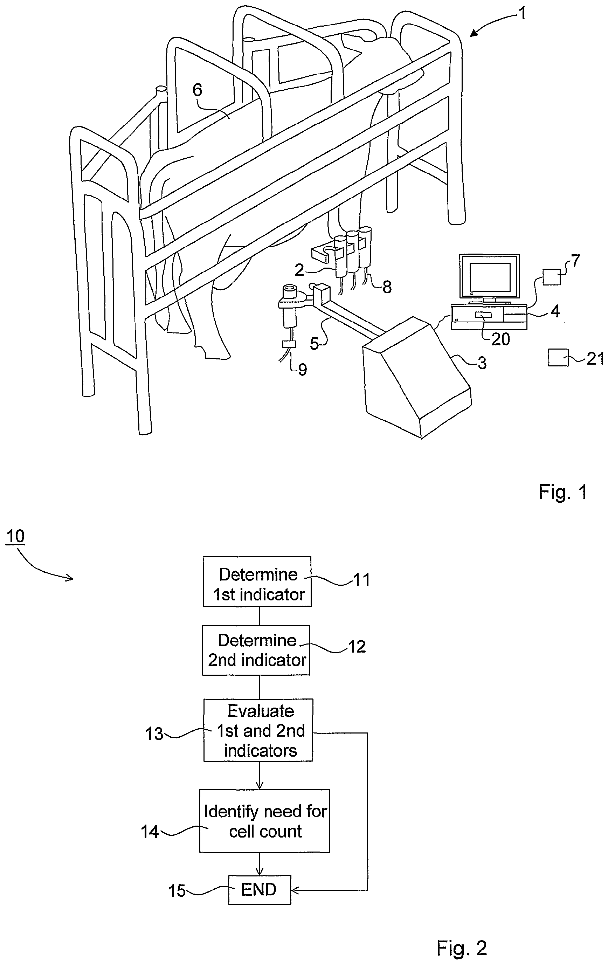

Figure 1 illustrates an automatic milking station or milking system in which the present invention may be implemented. Only parts relevant for understanding the present invention are mentioned in the following. It is realized that the automatic milking station 1 comprises other parts as well. The automatic milking station 1 is suitable for voluntary milking of cows, wherein the freely walking cow enters the milking station 1 in order to be milked on a voluntary basis. The milking system 1 comprises an automatic milking machine (not explicitly illustrated) including teat cups 2 connected to an end unit (not shown) by means of milk lines, part of which is shown at 8. The milking station 1 further comprises a milking robot or automatic handling device 3 including a processing and control device 4 and a robot arm 5. The milking robot 3 is arranged to automatically apply the teat cups 2 of the milking machine to the teats of a cow 6.

The processing and control device 4 is responsible for processing and controlling various actions in the milking station 1, such as activities in connection with the milking e.g. opening and closing of gates, controlling the milking machine and its handling device 3. The processing and control device 4, in the following denoted computer 4, typically comprises a microcomputer, suitable software and a database 7 including information about each of the cows milked by the milking machine, for example information about when the respective cow was last milked, when the cow was last fed, the milk production of the cow etc. The database thus contains data relating to the individual cows in the milking herd. In the figure the database 7 is shown as a separate entity, it may however be integrated with the computer 4.

As mentioned in the introductory part, somatic cell counting, quantified as cells per mL milk, is one of the most reliable methods available for determining the existence of mastitis in a herd of milking animals. However, a somatic cell count is rather expensive and requires some time in order to be performed. In accordance with the invention, the somatic cell count is therefore performed only after a need for it has been established.

In accordance with the invention, at least two mastitis indicating tests that are non-expensive and relatively simple to perform, but generally also less reliable, are first performed. In the following description such simple mastitis detection test are denoted initial mastitis detection tests. Examples of such initial mastitis detection tests include: conductivity tests; detection of colour changes in the milk; historical data, for example historical cell count results; measuring NAgase value, wherein Nagase is an enzyme (N-acetyl glucosaminidase) the concentration of which is indicative for mastitis; measuring the temperature of the milk; measuring the milk flow or the milk quantity from a specific teat; the cow's activity; the cow's nutritional status, i.e. how much fodder and/or water she has consumed.

The initial mastitis detection tests are thus preferably cheap and easy to perform and may consequently be performed often and on a regular basis. A conductivity test may for example be done at each milking occasion. The chances of an early detection of mastitis are thereby increased.

A mastitis infection can lead to damage of the gland's outer layer and such damage causes ions, such as sodium and chloride, to be released into the gland. The ions change the electrical potential of the milk. Measuring the changes in this electrical potential (the conductivity) of the milk can therefore indicate a possible mastitis infection. In a conductivity test, sensors, or electrodes, are arranged in the milk lines, for example in the milk lines near the teat cups 2. The electrodes are arranged to measure the conductivity of the milk, and an increased conductivity value gives an indication of mastitis. As mentioned, such conductivity test may be performed at each milking occasion and the results can be stored in the database 7. If the conductivity value is above a certain threshold, then this is taken as an indication of mastitis.

The mastitis infection often affects only one quarter of the udder, since each quarter of the udder is an independent gland. The conductivity test is therefore preferably performed for each teat. The conductivity test results from the different teats may be compared to each other in order to determine whether the cow has mastitis or not. In a healthy animal the conductivity values are about equal. The two initial mastitis detection tests may be the test results of conductivity values from two of the teats, for example the teats in the front or rear pair of teats.

Colour changes in the milk can be an indication of mastitis. The detection of colour changes in the milk can be done automatically in the milking station, for example by arranging sensor systems responding to a change in the visible or non-visible light transmission or reflection of the milk. Colour changes of the milk may thus be used as an indicator of mastitis, e.g. detection of abnormal udder secretions. Historical data, for example historical cell count results, may be used as one of the at least two first performed initial mastitis detection tests. All previously performed somatic cell count results for an individual cow are stored in the database 7 or the like. The data is accessible by means of the computer 4 connected to the automatic milking station 1. When the cow 6 enters the automatic milking station 1 she is identified and the relevant data for this particular cow 6 is retrieved. If the data obtained, for example previous cell count values, show unexpected results when compared to the current test data, this can be taken as an indication of incipient mastitis of the cow 6. For example, the variation in the cell count values of a particular cow may be taken as an indicator of mastitis. If a cow having rather uniform values over time suddenly has greatly deviating values this may cause suspicion of mastitis, whereas a cow usually having differing cell count values may be deemed not to require a cell count. Another example of a possible mastitis indicator is a steadily increasing trend of the test result values, and the results from such animal may then be observed more carefully or a visual inspection of her udder may be done. It is noted that other historical data may alternatively be used.

Other relevant information may alternatively be used as one of the indicators for performing a cell count test, such as for example the milk yield of the cow, the regularity of the milking intervals, her age, the number of calves she has had, which stage of the lactation cycle she is in etc. For example, the actual milk yield of the cow (i.e. the amount of milk actually obtained at a milking occasion) may be compared with the expected milk yield and if the actual milk yield is much lower than expected this is taken as an indication of mastitis. The expected milk yield may, for example, be determined based on historical milk yield for this particular cow. As another example, it is also possible to compare the actual milk yield from the individual teats with each other. The two front teats should have about the same milk yield, and the two back teats should also have about the same milk yield, else mastitis may be suspected. This comparison can also be made by comparing the milk yield from the left pair of teats to the milk yield from the right pair of teats.

The initial mastitis detection tests are preferably tests that can be made by means of sensors or other means included in the milking station 1. For example sensors for measuring the conductivity of the milk, sensors for sensing the colour changes of the milk, sensors for measuring the milk flow or sensors for measuring the temperature of the milk. An example of such sensor is indicated in figure 1 by reference numeral 9.

With reference to figure 2 the basic steps of the method 10 in accordance with the invention are illustrated. In steps 11 and 12 two initial tests indicative of mastitis are performed. The results of these two indicators are thereafter evaluated in step 13. The evaluation may be done in different ways, as will be described below. In step 14 a need for a somatic cell count is identified based on the result of the step of evaluation. That is, if the results of the two initial mastitis detection tests indicate such need, the need for a third test is identified. A decision about whether there is a need for a third mastitis indicating test, in the figure illustrated as a somatic cell count, is thus taken. If it is determined, in step 13, that there is no need for a cell count, the method ends in step 15. It is easy to increase the number of initial mastitis detection tests by simply adding indicator determination steps before the step of evaluating 13; there could for example be four initial mastitis indicating tests instead of only two. In the figure the steps 11 and 12 are shown to be performed in sequence; however, they may alternatively be performed in parallel, i.e. simultaneously. The method 10 in accordance with the invention may comprise the additional step of notifying a farmer about the need for a third mastitis indicating test, should such need be identified in step 14. The method 10 may also comprise the step of performing such third mastitis indicating test, which may be effectuated automatically upon such need being identified.

The step of evaluating the two or more initial mastitis indicating tests are described next. In the following, two initial mastitis detection tests are used for exemplifying the invention, but it is to be noted that three or even more such initial mastitis detection tests may be performed and then evaluated.

The first and second initial mastitis detection tests may be arranged to give a "Yes" or "No" result, or equivalently "1" or "0". If both initial mastitis detection tests indicate "Yes" then the cell count (or other third mastitis indicating test) is performed. If one of them indicates "Yes" and the other "No", a cell count may or may not be performed. Further initial mastitis detection tests may be performed if only one of the two first tests indicates mastitis. No cell count is however performed if both initial tests indicate "No".

As an example, if a conductivity test is used as one of the initial mastitis detection tests, and the conductivity value is above a threshold value that indicates mastitis, then the result is set to "Yes" (or "1"), else the result is set to "No" (or "0") .

The evaluation step can be based on a suitable mathematical algorithm. For example, different initial mastitis detection tests may be given different weight in the evaluation step and decision on whether to perform a cell count or not. The more reliable a test is believed to be, the more weight the test is given. If the two tests indicate different results, the decision may be based on the test having the highest weight.

Further, the results of a certain test may be affected by different factors and may also change over time; for example, factors such as the age of the cow or at which stage in the lactation cycle she is in may affect the results of some tests, as can her activity level on other tests. Such knowledge may be entered into the database 7 and be used in the evaluation. It is to be noted that threshold values may be set for each individual animal, as the value considered normal for a first animal may differ substantially from a value considered normal for another animal.

The evaluation step may be arranged to provide a mastitis indicator value as output. For example, two "Yes" (or 1 + 1) may be given the mastitis indicator value 1; one "Yes" and one "No" (or 1 + 0) may be suitably calculated based on the respective weight of the initial tests. For example: test 1 has weight 0,6 and indicates "No" ("0"), test 2 has weight 0,4 and indicates "Yes" ("1"), then a suitable mathematical algorithm could be

```
(weight of test 1) * (result of test 1) + (weight of
test 2) * (result of test 2), which in the example would give

(0,6 * 0 + 0,4 * 1) = 0,4
```

That is, the mastitis indicator value output from the evaluation step is 0,4. A cell count may, for example, be decided on if the mastitis indicator value is 0,5 or higher.

The activity level of a cow may be taken into consideration, that is, used as a first mastitis indicating test, wherein a low activity may be an indication of mastitis. The activity level of the cow goes down as she ages, and the activity level may therefore be weighted with her age. For example, test 1 may be her activity level and test 2 her age. A suitable mathematical algorithm could then be:

$$(\text{result of test 1})*(\text{result of test 2})=$$

$$= (\text{activity level})*(\text{age})$$

The result is then, as above, compared to a relevant threshold value.

In another example of mathematical algorithm, conductivity values from two teats may be used as the first and second initial mastitis detection tests. In the evaluation step 13, the conductivity values are compared. If there is a difference between the values and this difference is above some threshold, then this is taken as an indication of mastitis, whereupon the need for a third test is identified. The farmer may then be notified about such need and decide that a third test is to be performed.

From the above it is clear that any suitable way of combining the results of two or more initial mastitis indicating tests can be used for determining whether or not a third test, for example a somatic cell count, shall be performed. It is noted that other mathematical algorithms may be utilized. In some instances, an algorithm involving division operations may provide the most appropriate result.

It is to be noted that all steps of the method for detecting mastitis can be automated. The results from the first and second initial mastitis detection tests can be obtained automatically, for example from a suitable sensor such as probes for measuring conductivity, flow meters, sensors for sensing colour changes in the milk etc as previously mentioned. The results may then automatically be transmitted to the computer 4 for processing. A decision on whether or not to perform a cell count may then also be taken automatically, for example by executing a suitable mathematical algorithm in the computer 4.

The cell count itself is preferably performed in an automated manner on site, that is, instantly and in connection with the milking station 1. An instant cell counting is preferred in order to avoid having to await laboratory results. Further, the costs related to the cell count are thereby minimized. Since the manual handling of milk samples to be sent to the laboratories for the cell count, requires some time and attention of the farmer, such instant cell count is preferred. If it is determined, when the initial mastitis detection tests have been evaluated, that a further check in the form of a cell count should be done, a milk sample may be automatically sent to a sensor arrangement for counting the number of somatic cells in the sample.

However, if the cell count is not performed automatically, a display or other presentation device may be arranged for notifying the user that a cell count should be taken. For example, the first and the second mastitis indicating tests may be taken automatically, and if they show a need for a cell count test, then the farmer is informed about such need, for example by means of a display. He or she may then perform a manual cell count test. He may, for example, use a DeLaval Cell Counter (DCC), manufactured and sold by DeLaval International, for performing the third mastitis indicating test. The DCC is a portable and most convenient device for monitoring the somatic cell count level in a herd of milking animals.

The results of measurements and investigations which are not performed by sensors or other equipment connected to the milking station may of course be input manually to the computer 4 for processing.

In another embodiment of the invention the third indicator of mastitis is instead obtained by a bacterial investigation and/or an examination by a veterinary. In this embodiment, the first and/or the second indicator of mastitis is preferably a somatic cell count value. A cow having particularly high cell count values, or in other way alarming values, may need further investigation in order to determine which type of mastitis she suffers from and how best to treat her. Therefore, it may be necessary to perform a bacterial investigation or she may need to be examined by a veterinary.

Although it is possible to identify such need based on for example a single cell count measurement, it is preferred to base the identification on a more careful analysis. A single cell count measurement may be misleading as a cow can show large variations and sometimes rather high cell count values, e.g. 500 000 cells/ml, without there being a need to investigate her further. However, if her values are steadily low, a single high value may indicate a need for further investigation.

In a preferred embodiment, a cow is identified as a cow needing further investigation based on an analysis on historical data for that cow. The historical data may include the amount of milk that she has given, cell count values, reported medical treatments, lactation number, days in milk etc. By combining the historical data in a suitable manner, limits for acceptable or natural (expected) variation can be established. Limits for unacceptable or unnatural (unexpected) variation may correspondingly be established. If unacceptable deviations are determined to exist the cow is identified as a cow needing further investigation, and she may for example be put on a list for animals requiring the attention of a veterinary or on a list for animals requiring a bacterial investigation. A criterion for determining whether a cow is to be examined further or not is thus provided. The criterion may be based on cell count values in combination with other data, for example milk data and/or her medical history. As an example, if an expected measurement value C for a particular cow does not vary more than a calculated standard deviation $\sigma$ then it is determined that she does not need any special attention. However, if deviations larger than the standard deviation σ, or larger than a factor multiplied with the standard deviation σ, are determined, she is identified as a cow needing further examination.

The invention may be implemented in existing milking systems. Many milking systems comprise different sensors for sensing milking related variables, and the results from these existing sensors may be used in accordance with the present invention. The invention may then easily be implemented by adding suitable computer program product, for example stored on a computer readable medium such as a compact disc or floppy diskette, indicated schematically in figure 1 at 21. The method of the present invention can thus be implemented as a computer program product, which is loadable into the internal memory of the computer 4 of the milking system 1. The computer program product, indicated in figure 1 at reference numeral 20, comprises software code portions for performing the method when the product is run on the computer 4.

The invention thus also encompasses the computer program product 20. For example, the computer 4 comprises means for receiving the computer readable medium 21 and means for executing the computer program product 20 for carrying out the steps of: obtaining test results, for example from sensors of the milking system or from a database; comparing the results to relevant threshold values; processing the results, for example by utilizing a mathematical algorithm as described earlier; and for identifying a need for a third test. The computer program product 20 may also comprise software code portions for notifying a user about the need for a third test and/or for automatically performing such third test if need for it has been identified. Further related steps may also be implemented by means of the computer program product 20.

The invention is also related to software, or a computer program product directly loadable into the internal memory of a digital computer comprising software code portions for performing the steps in accordance with the invention when run on the computer 4.

Further, it is to be noted that the present invention may be implemented in different milking environments, for example in a milking rotary system, a parallel stall arrangement, herringbone stall, tandem stall configurations or any other arrangement.

Throughout the description a cow is used as an example of a milking animal when describing the invention. It is realized that the invention is applicable to other lactating animals as well.

Further, in the description a somatic cell count is used as an example of a third mastitis detection test, since such cell count is very reliable for detecting mastitis among milking animals. It should however be noted that the third test could be another reliable mastitis detection test. However, in a preferred embodiment, the invention provides a conditional somatic cell count mastitis test. If initial mastitis detecting tests show deviating results or both indicate the suspicion of mastitis in a cow, only then is the cell count performed.

While the present invention has been described in various embodiments it shall be appreciated that the invention is not limited to the specific features and details set forth, but is defined only by the appended patent claims.

**Claims**

**1.** A method (10) for detecting mastitis of a milking animal, the method comprising the steps of:

- automatically determining (11) a first indicator of mastitis,
- automatically determining (12) a second indicator of mastitis,
- automatically evaluating (13) said first indicator of mastitis and said second indicator of mastitis, and the method being **characterized by** further comprising the step of:
- automatically identifying (14) a need for a third indicator of mastitis based on the result from said step of evaluating (13).

**2.** The method for detecting mastitis as claimed in claim 1, wherein said step of evaluating (13) comprises applying a mathematical algorithm utilizing said first and second indicators in order to obtain a mastitis indicator value.

**3.** The method for detecting mastitis as claimed in any of claims 1-2, wherein said steps of automatically determining said first and second indicators of mastitis are performed automatically in the milking station during milking.

**4.** The method for detecting mastitis as claimed in any of claims 1-3, comprising the further step of, upon identifying (14) a need for a third indicator of mastitis, notifying a farmer about such need.

**5.** The method for detecting mastitis as claimed in claim 4, wherein said notification comprises a recommendation regarding which type of third indicator is preferred.

6. The method for detecting mastitis as claimed in any of claims 1-5, comprising the further step of, upon identifying (14) a need for a third indicator of mastitis, automatically determining a third indicator of mastitis.

7. The method for detecting mastitis as claimed in any of claims 1-6, wherein said third indicator of mastitis is determined by performing a somatic cell count, said third indicator of mastitis is the result of a bacterial investigation, or said third indicator of mastitis is the result of an examination by a veterinary.

8. The method for detecting mastitis as claimed in any of the preceding claims, wherein said step of determining (11) the first and/or second indicator of mastitis comprises one or more of: determining colour changes in the milk from said animal, determining a cell count value for said animal, retrieving historical cell count values for said animal, measuring the conductivity of the milk from said animal.

9. The method for detecting mastitis as claimed in any of claims 1-7, wherein said step of determining (11) the first indicator of mastitis comprises measuring the conductivity of the milk from a first teat of said animal, and said step of determining (12) the second indicator of mastitis comprises measuring the conductivity of the milk from a second teat of said animal.

10. The method for detecting mastitis as claimed in claim 9, wherein said step of evaluating (13) comprises comparing the conductivity value of milk from the first teat with the conductivity value of milk from the second teat, wherein if said comparison shows a difference that is above a threshold value, then a need for a third indicator of mastitis is identified.

11. The method for detecting mastitis as claimed in any of the preceding claims, wherein said steps of determining the first (11) and the second (12) indicators of mastitis are performed simultaneously.

12. A milking system (1) for milking a milking animal, said milking system (1) comprising a milking machine including teat cups (2) connected to an end unit by means of milk lines, and a computer (4), and comprising:

  - means for automatically obtaining a first and a second indicator of mastitis,
  - means for automatically evaluating said first and second indicator of mastitis, and

  the milking system being **characterized by** further comprising:

  - means for automatically identifying, based on the first and second indicators of mastitis, the need for a third indicator of mastitis.

13. The milking system as claimed in claim 12, wherein said computer (4) comprises a database (7) for storage of results of said first, second and third indicators of mastitis.

14. The milking system as claimed in any of claims 12-13, further comprising means for notifying a user about an identified need for a third mastitis indicating test.

15. The milking system as claimed in any of claims 12-14, wherein said means for obtaining the first and/or second indicator of mastitis comprises: sensors for measuring the conductivity of the milk from said animal, sensors for sensing colour changes in the milk from said animal or a sensor for determining a somatic cell count.

16. The milking system as claimed in any of claims 12-15, further comprising an on-line somatic cell counter.

17. A computer program product (20) loadable into the internal memory of a computer (4) of a milking station (1), comprising software code portions for carrying out the method as claimed in any of claims 1-11 when said product is run on said computer (4).

18. A computer program product (20) stored on a computer readable storage medium (21), comprising computer readable program code means for causing a computer (4) of a milking station (1) to carry out the method as claimed in any of claims 1-11.

**Patentansprüche**

1. Verfahren (10) zum Nachweis von Mastitis bei einem Milchtier, wobei das Verfahren die folgenden Schritte umfasst:

    - automatisches Ermitteln (11) eines ersten Mastitis-Indikators,
    - automatisches Ermitteln (12) eines zweiten Mastitis-Indikators,
    - automatisches Auswerten (13) des ersten Mastitis-Indikators und des zweiten Mastitis-Indikators, und wobei das Verfahren **dadurch gekennzeichnet ist, dass** es des Weiteren den folgenden Schritt umfasst:
    - automatisches Identifizieren (14) eines Bedarfs für einen dritten Mastitis-Indikator auf der Grundlage des Ergebnisses aus dem Schritt des Auswertens (13).

2. Verfahren zum Nachweis von Mastitis nach Anspruch 1, wobei der Schritt des Auswertens (13) das Anwenden eines mathematischen Algorithmus umfasst, welcher den ersten und den zweiten Indikator nutzt, um einen Mastitis-Indikatorwert zu erhalten.

3. Verfahren zum Nachweis von Mastitis nach einem der Ansprüche 1-2, wobei die Schritte des automatischen Ermittelns des ersten und des zweiten Indikators von Mastitis während des Melkens automatisch in der Melkstation durchgeführt werden.

4. Verfahren zum Nachweis von Mastitis nach einem der Ansprüche 1-3, das beim Identifizieren (14) eines Bedarfs für einen dritten Mastitis-Indikator den weiteren Schritt des Benachrichtigens eines Landwirts über einen solchen Bedarf umfasst.

5. Verfahren zum Nachweis von Mastitis nach Anspruch 4, wobei die Benachrichtigung eine Empfehlung in Bezug darauf umfasst, welche Art des dritten Indikators bevorzugt wird.

6. Verfahren zum Nachweis von Mastitis nach einem der Ansprüche 1-5, das beim Identifizieren (14) eines Bedarfs für einen dritten Mastitis-Indikator den weiteren Schritt des automatischen Ermittelns eines dritten Mastitis-Indikators umfasst.

7. Verfahren zum Nachweis von Mastitis nach einem der Ansprüche 1-6, wobei der dritte Mastitis-Indikator ermittelt wird, indem eine Zählung von somatischen Zellen durchgeführt wird, wobei der dritte Mastitis-Indikator das Ergebnis einer bakteriellen Untersuchung ist oder der dritte Mastitis-Indikator das Ergebnis einer Untersuchung durch einen Veterinär ist.

8. Verfahren zum Nachweis von Mastitis nach einem der vorstehenden Ansprüche, wobei der Schritt des Ermittelns (11) des ersten und/oder zweiten Mastitis-Indikators eines oder mehreres von Folgendem umfasst: Ermitteln von Farbveränderungen in der Milch von dem Tier, Ermitteln eines Zellenzählungswerts für das Tier, Abrufen von historischen Zellzählungswerten für das Tier, Messung der Leitfähigkeit der Milch von dem Tier.

9. Verfahren zum Nachweis von Mastitis nach einem der Ansprüche 1-7, wobei der Schritt des Ermittelns (11) des ersten Mastitis-Indikators das Messen der Leitfähigkeit der Milch von einer ersten Zitze des Tieres umfasst und der Schritt des Ermittelns (12) des zweiten Mastitis-Indikators das Messen der Leitfähigkeit der Milch von einer zweiten Zitze des Tieres umfasst.

10. Verfahren zum Nachweis von Mastitis nach Anspruch 9, wobei der Schritt des Auswertens (13) das Vergleichen des Leitfähigkeitswerts von Milch von der ersten Zitze mit dem Leitfähigkeitswert von Milch von der zweiten Zitze umfasst, wobei ein Bedarf für einen dritten Mastitis-Indikator identifiziert wird, wenn der Vergleich eine Differenz ergibt, die über einem Schwellenwert liegt.

11. Verfahren zum Nachweis von Mastitis nach einem der vorstehenden Ansprüche, wobei die Schritte des Ermittelns der ersten (11) und der zweiten (12) Indikatoren von Mastitis gleichzeitig durchgeführt werden.

12. Melksystem (1) zum Melken eines Milchtiers, wobei das Melksystem (1) eine Melkmaschine umfasst, die Melkbecher (2), welche mittels Milchleitungen mit einer Endeinheit verbunden sind, und einen Computer (4) einschließt, und welches umfasst:

    - Mittel zum automatischen Erhalten eines ersten und eines zweiten Mastitis-Indikators,

- Mittel zum automatischen Auswerten des ersten und des zweiten Mastitis-Indikators, und

wobei das Melksystem **dadurch gekennzeichnet ist, dass** es des Weiteren umfasst:

- Mittel zum automatischen Identifizieren des Bedarfs für einen dritten Mastitis-Indikator auf der Grundlage des ersten und des zweiten Mastitis-Indikators.

13. Melksystem nach Anspruch 12, wobei der Computer (4) eine Datenbank (7) zum Speichern von Ergebnissen des ersten, zweiten und dritten Mastitis-Indikators umfasst.

14. Melksystem nach einem der Ansprüche 12-13, das des Weiteren Mittel zum Benachrichtigen eines Benutzers über einen identifizierten Bedarf für einen dritten Mastitis anzeigenden Test umfasst.

15. Melksystem nach einem der Ansprüche 12-14, wobei die Mittel zum Erhalten des ersten und/oder zweiten Mastitis-Indikators umfassen: Sensoren zur Messung der Leitfähigkeit der Milch von dem Tier, Sensoren zur Erfassung von Farbveränderungen in der Milch von dem Tier oder einen Sensor zur Ermittlung einer Zählung von somatischen Zellen.

16. Melksystem nach einem der Ansprüche 12-15, das des Weiteren eine Online-Vorrichtung zur Zählung somatischer Zellen umfasst.

17. Computerprogrammprodukt (20), das in den internen Speicher eines Computers (4) einer Melkstation (1) geladen werden kann und Softwarecode-Abschnitte umfasst, mit denen sich das Verfahren nach einem der Ansprüche 1-11 durchführen lässt, wenn das Produkt auf dem Computer (4) ausgeführt wird.

18. Computerprogrammprodukt (20), das auf einem computerlesbaren Speichermedium (21) gespeichert ist und computerlesbare Programmcodemittel umfasst, um einen Computer (4) einer Melkstation (1) dazu zu bringen, das Verfahren nach einem der Ansprüche 1-11 durchzuführen.

**Revendications**

1. Procédé (10) de détection de la mammite chez un animal laitier, le procédé comprenant les étapes de :

- détermination automatique (11) d'un premier indicateur de la mammite,
- détermination automatique (12) d'un deuxième indicateur de la mammite,
- évaluation automatique (13) dudit premier indicateur de la mammite et dudit deuxième indicateur de la mammite, et le procédé étant **caractérisé en ce qu'**il comprend en outre l'étape de :
- identification automatique (14) d'un besoin d'un troisième indicateur de la mammite en fonction du résultat de ladite étape d'évaluation (13).

2. Procédé de détection de la mammite selon la revendication 1, dans lequel ladite étape d'évaluation (13) comprend l'application d'un algorithme mathématique utilisant lesdits premier et deuxième indicateurs pour obtenir une valeur d'indicateur de mammite.

3. Procédé de détection de la mammite selon l'une quelconque des revendications 1 et 2, dans lequel lesdites étapes de détermination automatique desdits premier et deuxième indicateurs de la mammite sont réalisées de manière automatique dans la station de traite pendant la traite.

4. Procédé de détection de la mammite selon l'une quelconque des revendications 1 à 3, comprenant l'étape supplémentaire, après identification (14) d'un besoin d'un troisième indicateur de la mammite, de notification d'un tel besoin à un agriculteur concernant.

5. Procédé de détection de la mammite selon la revendication 4, dans lequel ladite notification comprend une recommandation concernant le type préféré de troisième indicateur.

6. Procédé de détection de la mammite selon l'une quelconque des revendications 1 à 5, comprenant l'étape supplémentaire, après identification (14) d'un besoin d'un troisième indicateur de la mammite, de détermination automatique d'un troisième indicateur de la mammite.

**7.** Procédé de détection de la mammite selon l'une quelconque des revendications 1 à 6, dans lequel l'on détermine ledit troisième indicateur de la mammite en effectuant un comptage des cellules somatiques, ledit troisième indicateur de la mammite est le résultat d'une analyse bactérienne, ou ledit troisième indicateur de la mammite est le résultat d'un examen par un vétérinaire.

**8.** Procédé de détection de la mammite selon l'une quelconque des revendications précédentes, dans lequel ladite étape de détermination (11) du premier et/ou du deuxième indicateur de la mammite comprend au moins une des opérations suivantes : la détermination de changements de couleur du lait à partir dudit animal, la détermination d'une valeur de comptage cellulaire pour ledit animal, la récupération des valeurs historiques de comptage cellulaire pour ledit animal, la mesure de la conductivité du lait provenant dudit animal.

**9.** Procédé de détection de la mammite selon l'une quelconque des revendications 1 à 7, dans lequel ladite étape de détermination (11) du premier indicateur de la mammite comprend la mesure de la conductivité du lait provenant d'un premier trayon dudit animal, et ladite étape de détermination (12) du deuxième indicateur de la mammite comprend la mesure de la conductivité du lait provenant d'un deuxième trayon dudit animal.

**10.** Procédé de détection de la mammite selon la revendication 9, dans lequel ladite étape d'évaluation (13) comprend la comparaison de la valeur de conductivité du lait provenant du premier trayon à la valeur de conductivité du lait provenant du deuxième trayon, un besoin pour un troisième indicateur de la mammite étant identifié si ladite comparaison montre une différence supérieure à une valeur de seuil.

**11.** Procédé de détection de la mammite selon l'une quelconque des revendications précédentes, dans lequel lesdites étapes de détermination du premier (11) et du deuxième (12) indicateur de la mammite sont réalisées simultanément.

**12.** Système de traite (1) destiné à la traite d'un animal laitier, ledit système de traite (1) comprenant une machine de traite comprenant des gobelets trayeurs (2) reliés à une unité d'extrémité au moyen de lignes de traite, et un ordinateur (4), et comprenant :

- un moyen d'obtention automatique d'un premier et d'un deuxième indicateur de la mammite,
- un moyen d'évaluation automatique desdits premier et deuxième indicateurs de la mammite, et

le système de traite étant **caractérisé en ce qu'**il comprend en outre :

- un moyen d'identification automatique, en fonction du premier et du deuxième indicateur de la mammite, du besoin d'un troisième indicateur de la mammite.

**13.** Système de traite selon la revendication 12, dans lequel ledit ordinateur (4) comprend une base de données (7) destinée au stockage de résultats desdits premier, deuxième et troisième indicateurs de la mammite.

**14.** Système de traite selon l'une quelconque des revendications 12 et 13, comprenant en outre un moyen permettant de notifier à un utilisateur un besoin identifié d'un troisième test d'indication de la mammite.

**15.** Système de traite selon l'une quelconque des revendications 12-14, dans lequel ledit moyen d'obtention du premier et/ou du deuxième indicateur de la mammite comprend : des capteurs permettant de mesurer la conductivité du lait provenant dudit animal, des capteurs permettant de détecter des changements de couleur du lait provenant dudit animal ou un capteur permettant de déterminer un comptage de cellules somatiques.

**16.** Système de traite selon l'une quelconque des revendications 12-15, comprenant en outre un compteur de cellules somatiques en ligne.

**17.** Progiciel (20) chargeable dans la mémoire interne d'un ordinateur (4) d'une station de traite (1), comprenant des parties de code logiciel permettant de mettre en oeuvre le procédé selon l'une quelconque des revendications 1 à 11 lorsque ledit progiciel est exécuté sur ledit ordinateur (4).

**18.** Progiciel (20) stocké sur un support de stockage (21) lisible par ordinateur, comprenant un moyen de code de programme, lisible par ordinateur, destiné à amener un ordinateur (4) d'une station de traite (1) à exécuter le procédé selon l'une quelconque des revendications 1 à 11.

Fig. 1

Fig. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 01563609 A1